# EUROPEAN PATENT APPLICATION

(11) **EP 2 545 911 A1**
(43) Date of publication of application: **16.01.2013**
(21) Application number: 11173636.9
(22) Date of filing: 12.07.2011
(51) Int. Cl.: A61K 31/00, A61K 31/7004, A61K 31/7016, A61P 35/00

(54) **Use of Lipoteichoic Acid in the Treatment of Non-Encapsulated Cancer Cells**

(71) Applicant: Lunamed AG, 7000 Chur (CH)
(72) Inventor: Truog, Peter, 7000 Chur (CH)
(74) Representative: Peters, Hajo

(57) **Abstract**

The present invention refers to the treatment of non-encapsulated cancer cells using lipoteichoic acid especially also to a treatment following directly a medical event that actually or possibly liberated non-encapsulated cancer-cells into the body like surgical removal of cancer tissue or a biopsy.

## Description

### Field of the invention

The present invention refers to the treatment of non-encapsulated cancer cells using Lipoteichoic acid especially also to a treatment following directly a medical event that actually or possibly liberated non-encapsulated cancer-cells into the body like surgical removal of cancer tissue or a biopsy.

### Background of the invention

Cancer is a one of the most dreaded and feared diseases capturing the intense interest of nearly all levels of people but also causing enormous cost to health care. It is one of the most common causes of death in Western societies. The efforts to fight or to contain cancer are considerably and thus treatment of cancer is one of the most important targets of modern medicine. Even though a lot of causes for the initiation of cancer have been identified over the last decades, still the legitimate fear of a great number of people is contracting cancer which could happen to nearly everybody. In the light of this long need in society it was an overriding long-time goal and desire of society and researchers alike to identify means of reducing the risk of contracting cancer or even identifying a kind of prophylaxis or prophylactic vaccination against cancer. Thus a central goal of research and of this invention was to provide a means for cancer prophylaxis.

This invention surprisingly identified a way to fulfill this goal by the treatment of non-encapsulated cancer cells using Lipoteichoic acid.

Lipoteichoic acid (LTA) is a gram positive pathogen cell wall motif that allows the pathogen to be immunologically recognised by binding to Toll-like receptors on the cell surface. LTAs have been reported as having anti tumor activity (EP 135820; USP 4,678,773; A. Yamamoto et. al. 1985, Br. J. Cancer, 51, 739 - 742; and H. Usami et. al., Br. J. Cancer, 1988, 57, 70 - 73) ansd especially also WO 96/023896.

Accordingly, the invention is drawn to lipoteichoic acid as free acid or as any pharmaceutically acceptable salt or hydrate thereof for use in the treatment of non-encapsulated cancer cells.

By this method non-encapsulated cancer cells are removed or transformed into non-malignant forms or forms undergoing apostosis if treated by lipoteichoic acid. It came as a big surprise to the current inventors as they found out that when treating a patient having in a body fluid like blood or lymph or especially in body tissues like lymph nodes or solid tissue non-encapsulated cancer cells these cells completely disappeared. In further experiments it was found that these non-encapsulated (or not yet encapsulated) cancer cells were highly susceptible to the treatment with lipoteichoic acid especially if compared to encapsulated tumors (cancer cells) the established cancer/neoplasm. Still, it is just these non-encapsulated cells like also the non-encapsulated micro-tumors that form continuously in the body but who are the starting point of any development of cancer. Thus, controlling and removing these "young" non-encapsulated cancer cells is a way to control and contain development of cancer just from the outset. This treatment would thus also allow a prophylactic treatment of a person reducing the risk of contracting cancer by treating this person in fixed intervals (yearly, twice a year, quarterly or monthly) with the appropriate amount of lipoteichoic acid, thus blocking the development of cancer.

### Detailed Description:

Thus, as a central point, the invention related to lipoteichoic acid as free acid or as any pharmaceutically acceptable salt or hydrate thereof for use in the treatment of non-encapsulated cancer cells.

"Non-encapsulated cancer cells" are cancer/neoplastic cells not (yet) encapsulated by a capsule like a hyaluronic acid capsule. This includes cells not accumulated or aggregated with other cancer cells or to only accumulated or aggregated small groups of cancer cells not yet encapsulated and would also include microtumors. Microtumors of whom many are formed in the body at any given time is a small, not yet encapsulated population of malignant cells. This definition would not include real tumors ((encapsulated) cell lumps).

In a preferred embodiment of the invention, this treatment (according to the central point) is following a medical event that actually or possibly liberated non-encapsulated cancer-cells into the body. This is a special and very dangerous clinical moment as this liberated non-encapsulated cancer cells could be the starting point of a new development of cancer.

Thus, as a closely related second aspect this invention is also drawn to Lipoteichoic acid as free acid or as any pharmaceutically acceptable salt or hydrate thereof for use in the treatment of cancer or prophylaxis of cancer formation by administration following a medical event that actually or possibly liberated non-encapsulated cancer-cells into the body.

In any event, an often encountered risky manipulation of the body in which actually or possibly non-encapsulated cancer-cells are liberated into the body, is surgical removal of cancer tissue or a biopsy. Accordingly in embodiments according to the invention the mentioned medical event actually or possibly liberating non-encapsulated cancer-cells into the body is surgical removal of cancer tissue or a biopsy.

In preferred embodiments of the lipoteichoic acid used according to the invention, the treatment is following directly, preferably within 24 hours the medical event that actually or possibly liberated non-encapsulated cancer-cells into the body, like surgical removal of cancer tissue or a biopsy.

WO96/023896 A1 claim 10 mentions *"a method of treating cancer comprising administration of an antitumor effective amount of a lipoteichoic acid LTA-T or a physiologically acceptable salt thereof and optionally a monokine and*/*or hyaluronidase to a patient suffering from cancer, a tumor or a malignant cell thereof."* Such a treatment is fully disclaimed from the scope of protection of this application.

On one hand, this treatment is a treatment of a patient already suffering from cancer, or a tumor or a malignant cell thereof. This would accordingly not fall within the definition of prophylaxis of cancer formation. It would also not fall within the meaning of the "medical event actually or possibly liberating non-encapsulated cancer-cells into the body" or not into "following surgical removal of cancer tissue" as in this case the cancer would already have been removed or such an event is simply not mentioned and in addition it is a selected special treatment condition (e.g. after surgery and biopsy) not disclosed in this general and global treatment of cancer.

On the other hand, this treatment is also drawn to cancer or tumor, with both - and especially tumor being a cell lump (covered in a capsule) thus not being treatment of non-encapsulated cancer cells. In addition this general "tumor" unspecifically mentioned in WO96/02389 is also not a microtumor as microtumors are not tumors (encapsulated cell lumps) but the microtumor is just a small, not yet encapsulated population of malignant cells.

In addition, the "malignant cell thereof" (of the tumor) is a part of a tumor, a tumor being defined in liteature as an abnormal mass of tissue resulting from neoplasm. Tumor is clearly defined as a neoplasm that has formed a lump and thus is ancapsulated. Thus, as a tumor or cell lump has a capsule "malignant cell thereof" (of the tumor) are not falling under the definition of "non-encapsulated cancer cells".

The following provisos might apply either alone or in combination:
- with the proviso that the use for the treatment of a patient already suffering from cancer, or a tumor or a malignant cell thereof is excluded.
- with the proviso that the use for the treatment of a tumor or a malignant cell thereof is excluded.
- with the proviso that the non-encapsulated cancer cell is not or a tumor or a malignant cell thereof.

In a preferred embodiment of the lipoteichoic acid used according to the invention, the treatment is done by injection, including for example s.c. (subcutaneous), i.m. (intramuscular), i.v. (intravenous), i.a. (intra-arterial), intra-ocular, intra-pleural, intra-abdominal, or intra-thecal injection.

In another preferred embodiment of the lipoteichoic acid used according to the invention, the treatment is done by applying an amount of 0.1 to 10 µmol, 0.5 to 2.0 µmol or 1 µmol lipoteichoic acid applied in one or several applications per day.

In a preferred embodiment of the lipoteichoic acid used according to the invention, the treatment is repeated in regular intervals, including yearly, twice a year, quarterly, monthly or weekly.

Preferably the treatment with the lipoteichoic acid is done by injection (for example s.c., intra-pleural, intra-abdominal, or intra-thecal injection) of 0.5 to 2.0 µmol or 1 µmol of lipoteichoic acid (e.g. in 1µmol/ml). Preferably the lipoteichoic acid in such an injectable formulation is preferably dissolved in a physiologically acceptable aqueous medium, most preferably in physiological saline (NaCl 0.9%). The injectable formulation may be used for single or repeated (from 1 to 10 times per day) injections.

The medicament or pharmaceutical formulation for use in the treatment of the present invention may thus in general be administered parentally in combination with conventional injectable liquid carriers, such as water or suitable alcohols (injectable formulations). Conventional pharmaceutical excipients for injection, such as stabilizing agents, solubilizing agents, and buffers, may be included in such injectable compositions.

In general though, the treatment with the lipoteichoic acid can be done with any pharmaceutical formulation or medicament comprising lipoteichoic acid and optionally one or more pharmaceutically acceptable excipients. The medicament or pharmaceutical formulation for use in the treatment according to the present invention can be of any form suitable for the application to humans and/or animals, preferably humans including infants, children and adults and can be produced by standard procedures known to those skilled in the art. The medicament or pharmaceutical formulation can be produced by standard procedures known to those skilled in the art, e.g. from the table of contents of "Pharmaceutics: The Science of Dosage Forms", Second Edition, Aulton, M.E. (ED. Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modern Pharmaceutics", Fourth Edition, Banker G.S. and Rhodes C.T. (Eds.) Marcel Dekker, Inc. New York 2002 y "The Theory and Practice of Industrial Pharmacy", Lachman L., Lieberman H. And Kanig J. (Eds.), Lea & Febiger, Philadelphia (1986). The respective descriptions are hereby incorporated by reference and form part of the disclosure. The composition of the medicament may vary depending on the route of administration.

Medicaments or pharmaceutical formulation for use in the treatment according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, granules, capsules, lozenges, aqueous or oily solutions, suspensions, emulsions, or dry powdered forms suitable for reconstitution with water or other suitable liquid medium before use, for immediate or modified or controlled release. The multiparticulate forms, such as pellets or granules, may e.g. be filled into a capsule, compressed into tablets or suspended in a suitable liquid.

Suitable controlled release formulations, materials and methods for their preparation are known from the prior art, e.g. from the table of contents of "Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000); "Controlled Drug Delivery", Vol, I, Basic Concepts, Bruck, S.D. (Ed.), CRD Press Inc., Boca Raton (1983) y de Takada, K. and Yoshikawa, H., "Oral Drug Delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encyclopedia of Controlled Drug Delivery, Mathiowitz, E. (Ed.), John Wiley & Sons, Inc., New York (1999), Vol. 2, 698-728. The respective descriptions are hereby incorporated by reference and form part of the disclosure.

Medicaments or pharmaceutical formulation for use in the treatment according to the present invention may also comprise an enteric coating, so that their dissolution is dependent on pH-value. Due to said coating the medicament may pass the stomach undissolved and the respective pharmaceutical composition according to the central aspect of the invention is liberated in the intestinal tract. Preferably the enteric coating is soluble at a pH value of 5 to 7.5. Suitable materials and methods for the preparation are known from the prior art. Typically, these medicaments for use in the treatment according to the present invention may contain 1-60 % by weight of lipoteichoic acid and 40-99 % by weight of one or more auxiliary substances (additives/excipients).

The pharmaceutical combination for use in the treatment of the present invention or medicament or pharmaceutical formulation for use in the treatment of the invention may also be administered topically or via a suppository.

"Lipoteichoic acids" - also abbreviated as "LTA" used herein is an inclusive term including the a) lipoteichoic acids (LTA) There are many lipoteichoic acids known which form a major part of the cell wall of gram-positive bacteria. The known lipoteichoic acids include synthetic, semisynthetic and naturally occurring lipoteichioic acids. A very broad introduction on the synthesis of as well as on synthetic lipoteichoic acids can be found in Pedersen, Christian Marcus; Schmidt, Richard R. (Edited by Moran, Anthony P), Microbial Glycobiology (2009), 455-476 with all lipoteichoic acids described therein herewith included by reference in this application as examples of the lipoteichoic acids to be used. A further overview of the lipoteichoic acids - divided in Types I to IV - can be found in Greenberg et al., Infect Immun. 1996 Aug;64(8):3318-25. All lipoteichoic acids Type I, Type II, Type III and Type IV as well as all those described therein (like e.g. in tables 1 or 2) herewith included by reference in this application as examples of the lipoteichoic acids to be used.

LTAs were isolated from e. g. Lactobacillus helveticus (NCIB 8025), Lactobacillus fermenti (NCTC 6991), Streptococcus faecalis, 39, Streptococcus lactis (ATCC 9936), Streptococcus mutans, AHT (A. J. Wicken et al, 1975), and Streptococcus pyogenes SV strain (ATCC 21059) (EP 135 820, USP 4,678,773, H. Usami et. al. 1985).

In a preferred embodiment of the lipoteichoic acid for use according to the invention the lipoteichoic acid is a (general) lipoteichoic acid. The (general) lipoteichoic acids as defined in this application are comprising:
a) a linear, hydrophilic 1,3-connected glycero-phophate chain - optionally substituted with D-alanine or other molecules -,
b) the gylcerophosophate chain being covalently bound through a phosphodiester to
c) a glycolipid, preferably a glyceroglycolipid.

Lipoteichoic acids falling under this definition according to this invention are called "(general) lipoteichoic acids" especially hereinafter.

The glycolipid acts as an anchor in the cell membrane. The glycolipids are by definition of the invention lipids of the membrane in which one or more mono- or oligosaccharids are bound via a glycosidic bond to a lipid molecule. The lipid molecule is consisting of fatty acids being bound by an ester bond to a glycerol or by an amide bond to a sphingosine.

In one preferred embodiment of the lipoteichoic acid for use according to the invention relating to a (general) lipoteichoic acid the glycolipid is a glyceroglycolipid in which one or more mono- or oligosaccharids are bound via a glycosidic bond to a lipid molecule consisting of fatty acids being bound by an ester bond to the glycerol. Preferably the glyceroglycolipid comprises a diacylglycerol with two fatty acid chains of 10 to 20 carbon atoms each being bound by an ester bond to the glycerol; and/or preferably the fatty acid chains are linear or branched and non-substituted and/or have a length of 10 to 20 carbon atoms each, most preferably 12, 14, 16 or 18 carbon atoms.

Most (general) lipoteichoic acids would be covered by the general formula I and its substituents as described and defined below and some preferred embodiments are described below, but some are unusual. These unusual include the lipoteichoic acid from *Streptococcus pneumoniae* (see below) whose synthesis was recently described by Pedersen et al., in Angew. Chem. (2010), 122,1 - 7. 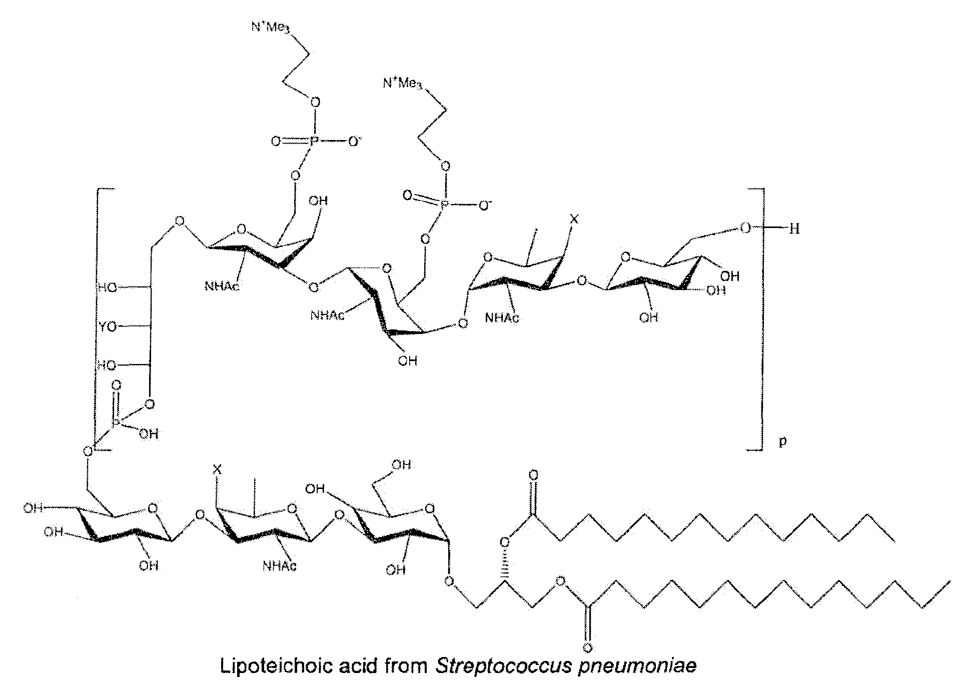 wherein
in one case X is NH₃⁺, or NHAc; Y is H, D-Ala, or α-GalNAc; and p is up to 8;
in the other case X is NH₃⁺; Y is H; and p is 1.

In a preferred embodiment of the lipoteichoic acid for use according to the invention the lipoteichoic acid is a lipoteichoic acid according to general formula I 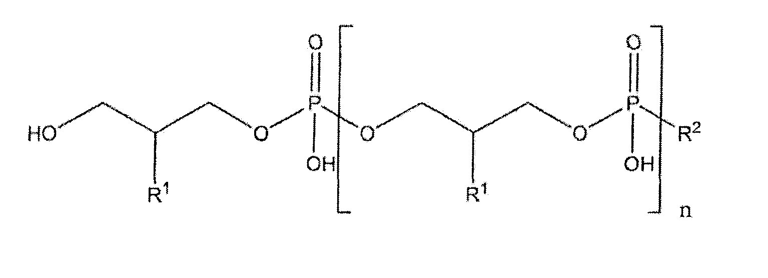 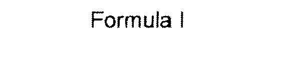 wherein
the mean value of n is 8 to 40,
R¹ is either OH or O-D-alanyl and
R² is either 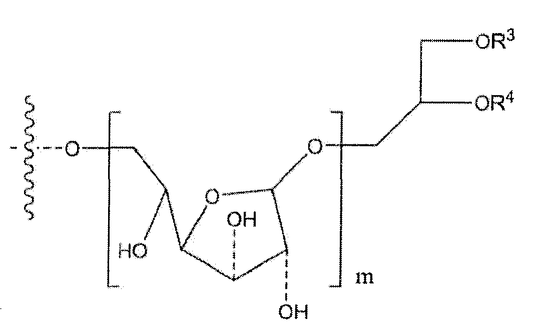
or its tautomeric equivalent 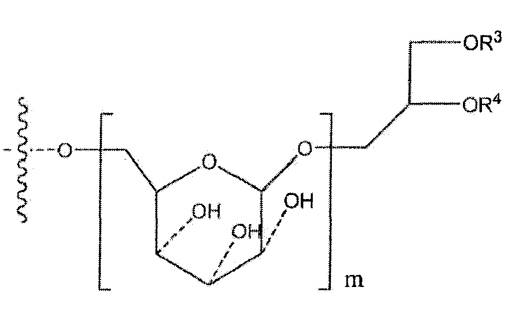
with R³ and R4 independently from one another being residues of saturated or unsaturated, unsubstituted fatty acids with 10 to 20 carbon atoms and m being selected from 1 to 3,
with the compounds of formula I being present either in free form or as physiologically acceptable salt; in form of any of their structural isomers including mixtures thereof, preferably in pure enantiomeric or diastereomeric form or any mixture thereof including racemic mixtures.

In one even more preferred embodiment of the lipoteichoic acid for use according to the invention the lipoteichoic acid is a lipoteichoic acid according to general formula I 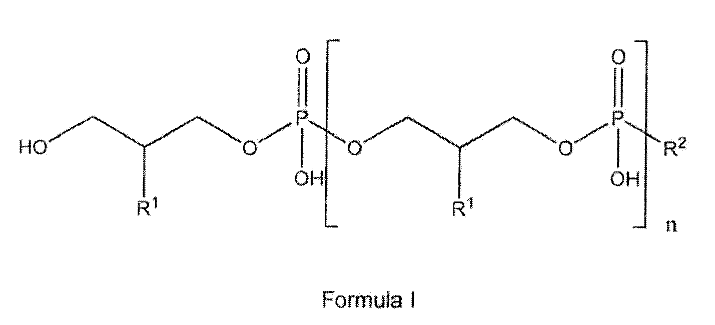 wherein
the mean value of n is 9,
R¹ is either OH or O-D-Alanyl and
R² is 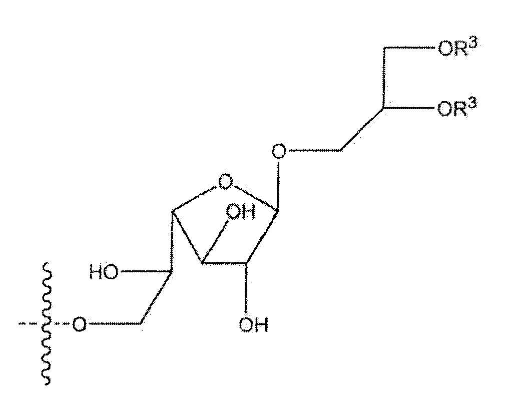
with R³ being residues of saturated or unsaturated fatty acids with 12, 14, 16 or 18 carbon atoms,
with the compounds of formula I being present either in free form or as physiologically acceptable salt; in form of any of their structural isomers including mixtures thereof, preferably in pure enantiomeric or diastereomeric form or any mixture thereof including racemic mixtures. These compounds defined above as well as those described by general formula Ia, Ib, Ic or Id and their defined radicals below (as well as any lipoteichoic acid to be isolated from the Streptococcus sp. PT strain DSM 8747) are defined in this invention as "lipoteichoic acid of the invention".

In a further embodiment of the lipoteichoic acid for use according to the invention the lipoteichoic acid is a purified lipoteichoic acid isolated or isolatable from the Streptococcus sp. PT strain DSM 8747, preferably containing a beta-galactofuranosyl(1-3)glycerol-di-ester moiety.

In a preferred embodiment of the lipoteichoic acid for use according to the invention the lipoteichoic acid is a lipoteichoic acid according to any of general formulas Ia, Ib, Ic or Id 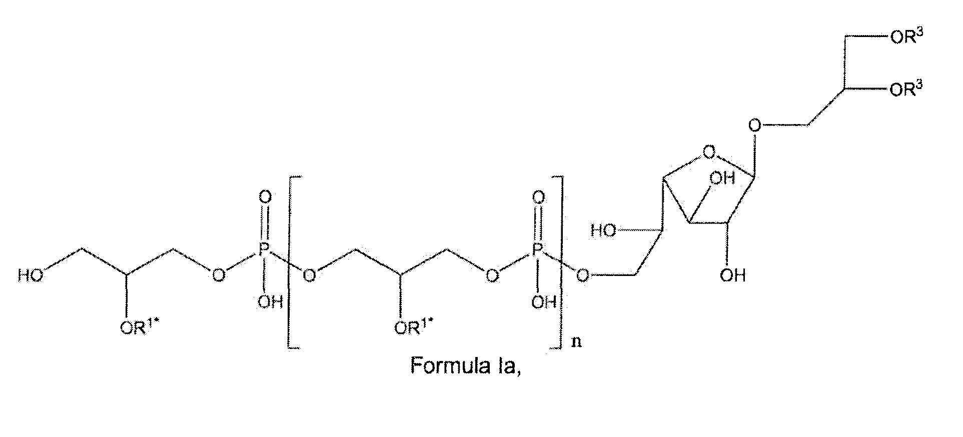 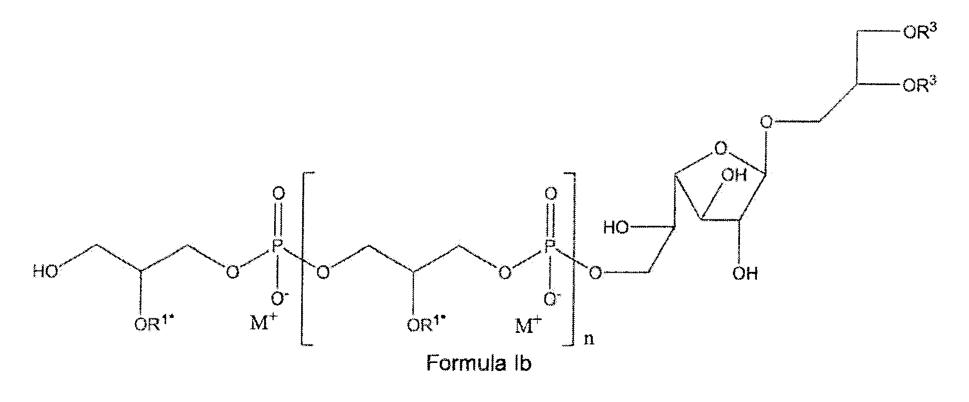 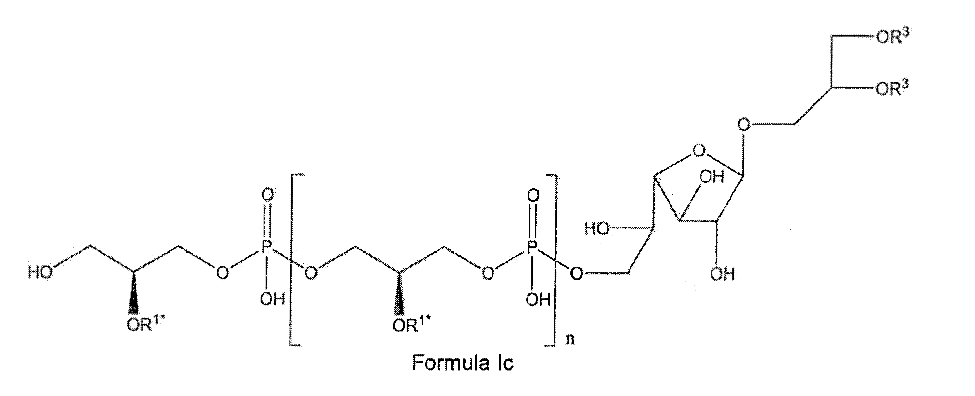 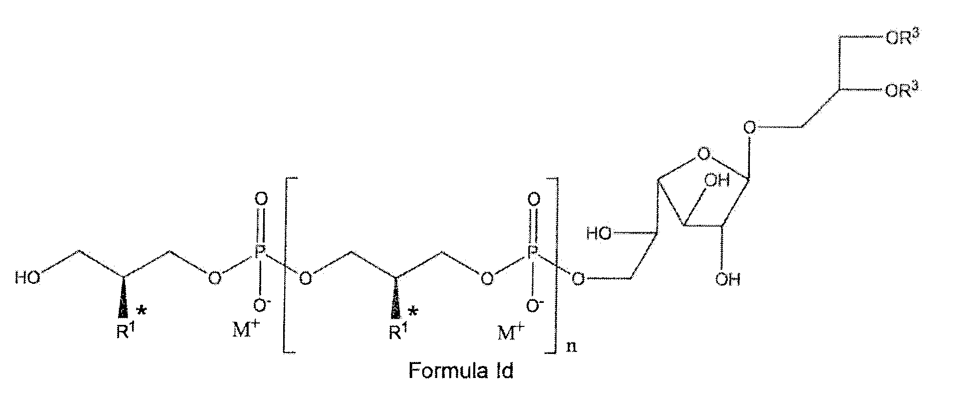 with
n having a mean value of 9, preferably being 6 to 12 while having a mean value of 9,
R^{1*} being either H or D-alanyl,
R³ being residues of saturated or unsaturated fatty acids with 12, 14, 16 or 18 carbon atoms, and
M⁺ being selected from positively charged ions, such as alkali metal ions or positively charged primary, secondary, tertiary or quaternary ammonium ions, preferably M⁺ being a sodium ion.

In a preferred embodiment of the lipoteichoic acid for use according to the invention the lipoteichoic acid is a purified lipoteichoic acid isolated from bacteria of the genus *streptococcus,* preferably from *streptococcus sp.,* most preferably from *streptococcus sp.* (DSM 8747). One example of such an isolated lipoteichoic acid is lipoteichoic acid T (LTA-T).

Lipoteichoic acids - especially the lipoteichoic acid T (LTA-T) - are described in the PCT-application WO 96/23896 together with its antitumor cholesterol-lowering activity and ways of isolating the LTA-T. The whole disclosure of WO06/23896 is included in this application by way of reference.

LTA-T is a gram positive cell wall motif that allows it to be immunologically recognised by binding to Toll-like receptors on the cell surface. LTA-T is described in US 6,114,161 for use as an antitumour preparation. All aspects of LTA-T from US 6,114,161 are incorporated herein by reference.

Preferably, the "lipoteichoic acid" is lipoteichoic acid T (LTA-T). Thus, in one very preferred embodiment the lipoteichoic acid for use according to the invention is lipoteichoic acid T (LTA-T).

Lipoteichoic acid or especially lipoteichoic acid T (LTA-T) may form salts with other (positively) charged ions, in particular physiologically acceptable salts, such as alkali metal or alkaline earth metal salts, also heavy metal salts, such as zinc or iron salts, or primary, secondary, tertiary or quaternary ammonium salts (acid addition salts) . Such other salts are e. g. potassium, calcium, ammonium, mono-, di-, tri- or tetra-lower alkyl-, like methyl- or ethyl-, or methyl-ethyl, propyl- or butylammonium salts. A prefered salt of LTA-T is the sodium salt.

In another embodiment of lipoteichoic acid for use according to the invention the lipoteichoic acid according to general formula I is a lipoteichoic acid of *staphylococcus aureus* (below): 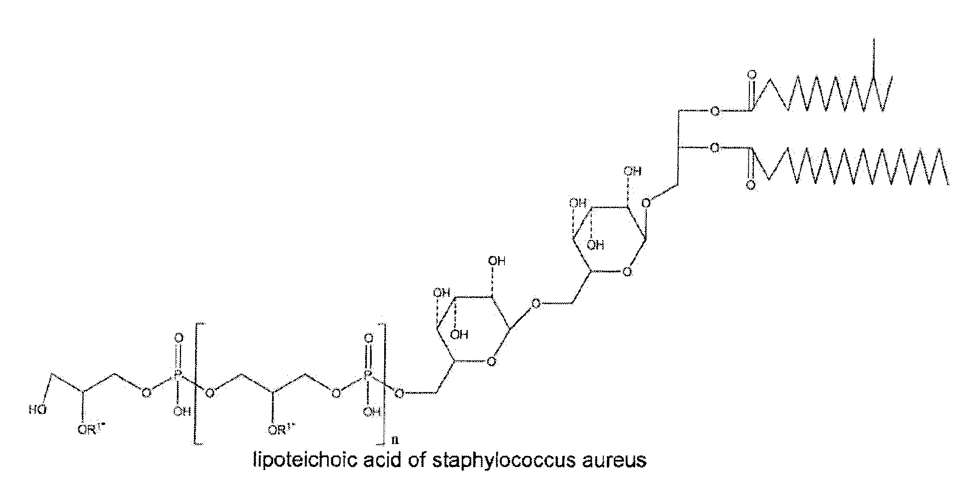 , wherein X is H or D-ala and the mean value of "n" is 16 to 40.

"Hydrate" is defined as a substance/joint mixture according to the invention containing water. Thus, it may be a crystalline salt with one or more water molecules in the crystalline structure or also amorphous containing water molecules.

"Solvate" is defined as a substance/joint mixture according to the invention containing a further solvent. One most prominent example of the solvents is an alcohol like ethanol or methanol thus leading to methanolates or ethanolates. Thus, it may be a crystalline salt with one or more alcohol molecules in the crystalline structure or also an amorphous form containing alcohol molecules.

In another preferred embodiment of the invention the treatment is done with a pharmaceutical composition comprising at least one lipoteichoic acid as free acid or as any pharmaceutically acceptable salt or hydrate thereof and at least one D-amino acid as free acid or as any pharmaceutically acceptable salt or hydrate thereof. Preferably this lipoteichoic acid is any of the lipoteichoic acids set out above and most preferably is LTA-T. The D-amino acid may be any D-amino acid but preferably is D-alanine. This pharmaceutical composition is preferably an injectable formulation comprising (e.g. 1 µmol) Lipoteichoic acid (LTA-T) and (e.g. 50 mg) D-amino acid (D-alanine).
This inventive concept would also apply to a related aspect of the invention wherein the treatment is done by applying (e.g by (e.g. s.c.) injection of an injectable formulation) a pharmaceutical composition comprising at least one lipoteichoic acid (LTA-T) as free acid or as any pharmaceutically acceptable salt or hydrate thereof and by applying separately (e.g orally in an oral pharmaceutical formulation) before or after or simultaneously at least one D-amino acid (D-alanine) as free acid or as any pharmaceutically acceptable salt or hydrate thereof. This related aspect would naturally be also the subject of a method of treatment of cancer.

One very preferred aspect of the invention would also encompass the lipoteichoic acid for the use according to the invention in which the cancer would be in the form of non-encapsulated cancer cells but also in which the non-encapsulated cancer cells (or also the tissue (immediately) surrounding them) are in an inflammatory state. This would include a treatment after induction of inflammation in the non-encapsulated cancer cells. This would also include a treatment in which the non-encapsulated cancer cells are pro-inflammatorically co-treated. All of this would thus also encompass a method of treatment or better of prophylaxis against (by lowering the risk of getting) cancer by providing to a patient in need thereof (at risk of suffering from cancer in the future (due to non-encapsulated cancer cells) or accordingly just having undergone a surgical cancer treatment or biopsy) a sufficient amount of lipoteicoic acid, in which the patient is further co-treated (like a local treatment e.g. at or around the area of surgery of biopsy) in a pro-inflammatory way. This pro-inflammatory co-treatment might occur by (X-ray; iodizing) radiation or by application of at least one pro-inflammatory substance like pro-inflammatory interleukins. By this way the chances of a successful prophylactic treatment are increased.

A last aspect of the invention would further encompass a method of treatment or better of prophylaxis against (by lowering the risk of getting) cancer by providing to a patient in need thereof (at risk of suffering from cancer in the future (due to non-encapsulated cancer cells) or accordingly just having undergone a surgical cancer treatment or biopsy) a sufficient amount of lipoteicoic acid, in which the patient is further co-treated (like a local treatment e.g. at or around the area of surgery of biopsy) in a pro-inflammatory way and in which the treatment with lipoteichoic acid is done with a pharmaceutical composition comprising at least one lipoteichoic acid as free acid or as any pharmaceutically acceptable salt or hydrate thereof and at least one D-amino acid as free acid or as any pharmaceutically acceptable salt or hydrate thereof. Preferably this lipoteichoic acid is any of the lipoteichoic acids set out above and most preferably is LTA-T. The D-amino acid may be any D-amino acid but preferably is D-alanine. This pharmaceutical composition is preferably an injectable formulation comprising (e.g. 1 µmol) lipoteichoic acid (LTA-T) and (e.g. 50 mg) D-amino acid (D-alanine). The pro-inflammatory co-treatment might occur by (X-ray; iodizing) radiation or by application of at least one pro-inflammatory substance like pro-inflammatory interleukins.
The last aspect of the invention would also apply to a related aspect of the invention wherein the treatment is done by applying (e.g. by (e.g. s.c.) injection of an injectable formulation) a pharmaceutical composition comprising at least one lipoteichoic acid (LTA-T) as free acid or as any pharmaceutically acceptable salt or hydrate thereof and by applying separately (e.g orally in an oral pharmaceutical formulation) before or after or simultaneously at least one D-amino acid (D-alanine) as free acid or as any pharmaceutically acceptable salt or hydrate thereof. This last aspect would naturally be also the subject of a method of treatment of cancer.

The present invention is illustrated below with the help of the following examples. These illustrations are given solely by way of example and do not limit the invention.

### EXAMPLES:

### Example 1: Pharmaceutical Formulation

The purified LTA-T in 0.05M sodiumacetate pH 4.7 (prepared as set out in WO 1996/023896) is dialysed extensively against physiological NaCl solution (0.9%) and the volume is adjusted to 1 umol LTA-T (based on the phosphorus content) within 1 ml of physiological saline. After filtration of the solution through a filter membrane (Millipore 0.22um), 1 ml aliquots of the filtrate are placed in sterilized vials under sterile conditions. These vials contain 1 micromol/ml LTA-T phosphorus and are used subcutaneously for therapeutical purposes.

## Claims

1. Lipoteichoic acid as free acid or as any pharmaceutically acceptable salt or hydrate thereof for use in the treatment of non-encapsulated cancer cells.

2. The Lipoteichoic acid according to claim 1, wherein the treatment is following a medical event that actually or possibly liberated non-encapsulated cancer-cells into the body.

3. Lipoteichoic acid as free acid or as any pharmaceutically acceptable salt or hydrate thereof for use in the treatment of cancer or prophylaxis of cancer formation by administration following a medical event that actually or possibly liberated non-encapsulated cancer-cells into the body.

4. The Lipoteichoic acid according to any of claims 2 or 3, wherein the medical event is surgical removal of cancer tissue or a biopsy.

5. The lipoteichoic acid according to any of claims 2 to 4, wherein the treatment is following directly, preferably within 24 hours the medical event that actually or possibly liberated non-encapsulated cancer-cells into the body, like surgical removal of cancer tissue or a biopsy.

6. The lipoteichoic acid according to any of claims 1 to 5, wherein the treatment is done by injection, including parenteral injection, including, s.c., i.m., i.v., i.a., intra-ocular, intra-pleural, intra-abdominal, or intra-thecal injection.

7. The lipoteichoic acid according to any of claims 1 to 6, wherein the treatment is done by applying an amount of 0.1 to 10 µmol, 0.5 to 2.0 µmol or 1 µmol lipoteichoic acid applied in one or several applications per day.

8. The lipoteichoic acid according to any of claims 1 to 7, wherein the treatment is repeated in regular intervals, including yearly, twice a year, quarterly, monthly or weekly.

9. The Lipoteichoic acid according to any of claim 1 to 8 comprising:
a) a linear, hydrophilic 1,3-connected glycero-phophate chain - optionally substituted with D-alanine or other molecules -,
b) the gylcerophosophate chain being covalently bound through a phosphodiester to
c) a glycolipid, preferably a glyceroglycolipid.

10. The Lipoteichoic acid according to claim 9 wherein the glycolipid is a glyceroglycolipid in which one or more mono- or oligosaccharids are bound via a glycosidic bond to a lipid molecule consisting of fatty acids being bound by an ester bond to the glycerol.

11. The Lipoteichoic acid according to claim 10 wherein the glyceroglycolipid comprises a diacylglycerol with two fatty acid chains of 10 to 20 carbon atoms each being bound by an ester bond to the glycerol,
and/or wherein the fatty acid chains being linear or branched and non-substituted and/or having a length of 10 to 20 carbon atoms each, most preferably of 12, 14, 16 or 18 carbon atoms.

12. The Lipoteichoic acid according to any of claims 1 to 8 being a structure according to general formula I 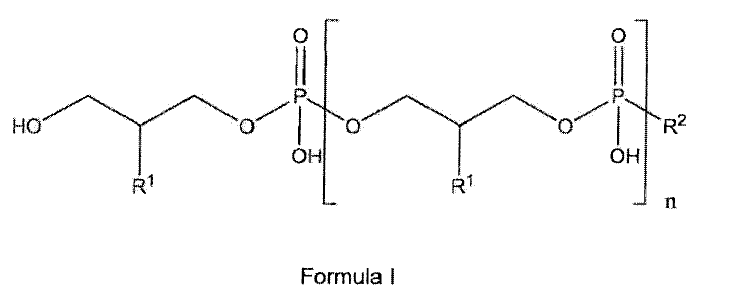 wherein
the mean value of n is 8 to 40,
R¹ is either OH or O-D-alanyl and
R² is either 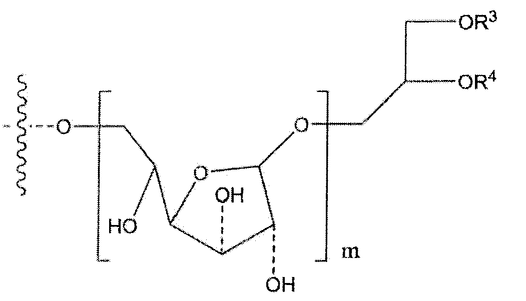
or its tautomeric equivalent 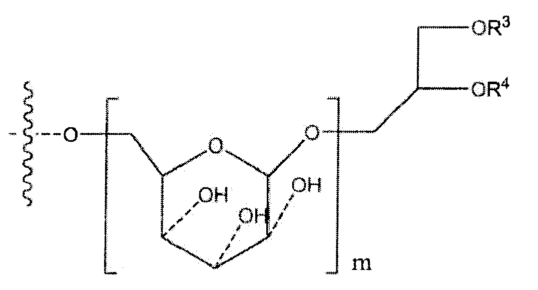
with R³ and R4 independently from one another being residues of saturated or unsaturated, unsubstituted fatty acids with 10 to 20 carbon atoms and m being selected from 1 to 3,
with the compounds of formula I being present either in free form or as physiologically acceptable salt; in form of any of their structural isomers including mixtures thereof, preferably in pure enantiomeric or diastereomeric form or any mixture thereof including racemic mixtures;
preferably being a structure according to general formula I 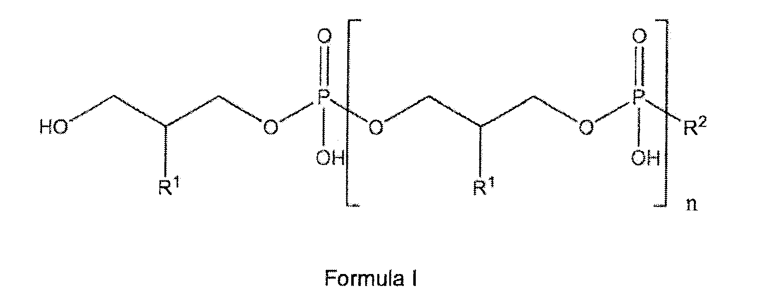 wherein
the mean value of n is 9,
R¹ is either OH or O-D-Alanyl and
R² is 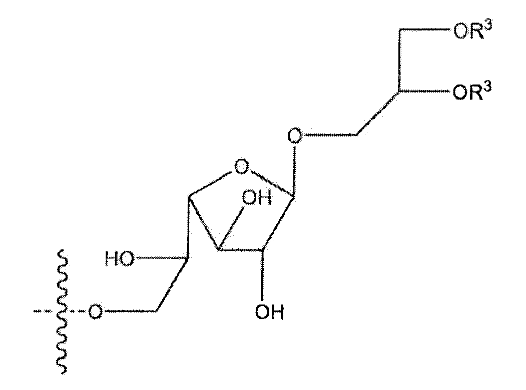
with R³ being residues of saturated or unsaturated fatty acids with 12, 14, 16 or 18 carbon atoms,
with the compounds of formula I being present either in free form or as physiologically acceptable salt; in form of any of their structural isomers including mixtures thereof, preferably in pure enantiomeric or diastereomeric form or any mixture thereof including racemic mixtures.

13. The Lipoteichoic acid according to any of claims 1 to 8 being a purified lipoteichoic acid isolated or isolatable from the Streptococcus sp. PT strain DSM 8747, preferably containing a beta-galactofuranosyl(1-3)glycerol-di-ester moiety.

14. The Lipoteichoic acid according to any of claims 1 to 8 being a compound according to any of general formulas la, Ib, Ic, or Id 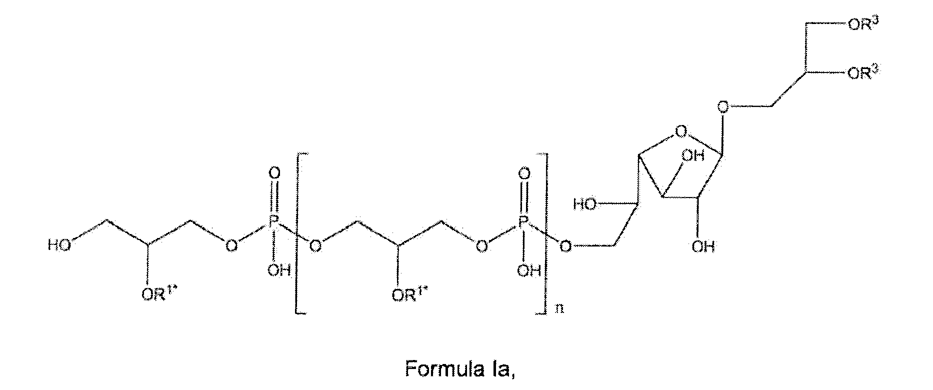 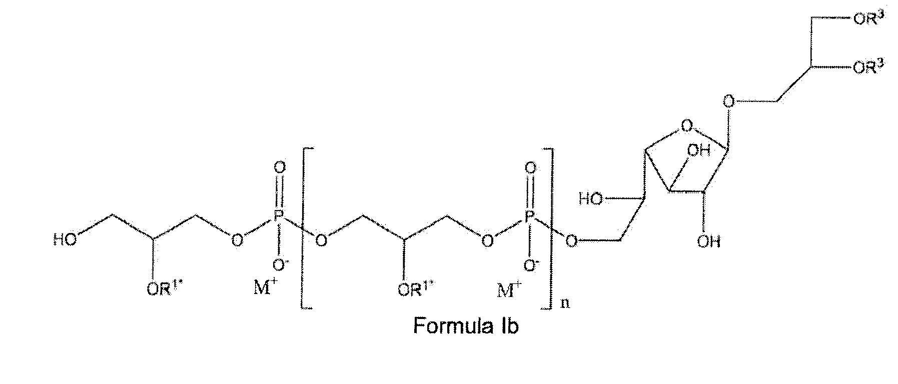 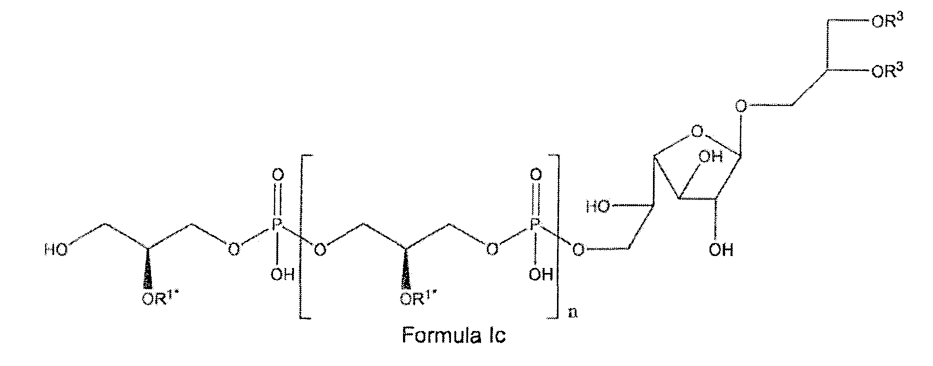 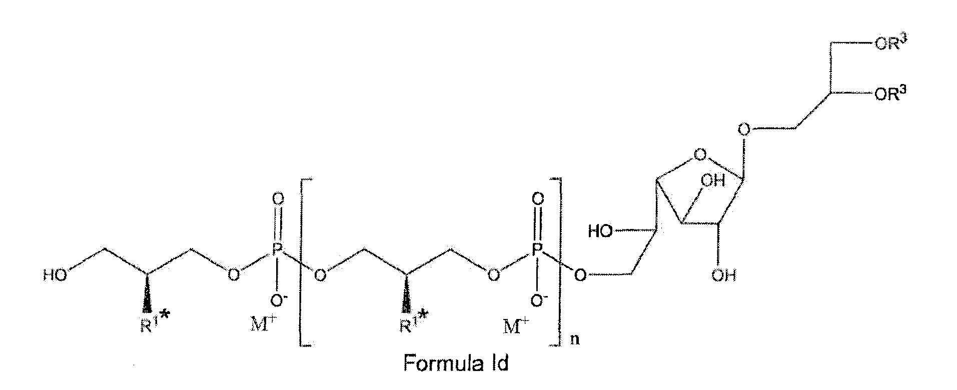 with
n having a mean value of 9, preferably being 6 to 12 while having a mean value of 9,
R^{1*} being either H or D-alanyl,
R³ being residues of saturated or unsaturated fatty acids with 12, 14, 16 or 18 carbon atoms, and
M⁺ being selected from positively charged ions, such as alkali metal ions or positively charged primary, secondary, tertiary or quaternary ammonium ions, preferably M⁺ being a sodium ion.

15. The Lipoteichoic acid according to any of claims 1 to 8 being LTA-T as a free acid or as a pharmaceutically acceptable salt or hydrate thereof, especially being LTA-T as a free acid or as a sodium salt.
